# FASCICULE DE BREVET EUROPEEN

(11) **EP 0 877 785 B1**
(45) Date de publication et mention de la délivrance du brevet: **02.01.2002**
(21) Numéro de dépôt: 96933510.8
(22) Date de dépôt: 10.10.1996
(51) Int. Cl.: C11B 9/02, A61K 7/48, A61K 35/78

(54) **NOUVEAUX EXTRAITS DE PLANTES, LEUR PROCEDE DE FABRICATION ET LEUR UTILISATION DANS LES PRODUITS COSMETIQUES**
NEUARTIGE PFLANZENEXTRAKTE, HERSTELLUNGSMETHODE FÜR DIESELBEN SOWIE VERWENDUNG IN KOSMETIKA
NOVEL PLANT EXTRACTS, PREPARATION METHOD THEREFOR AND USE THEREOF IN COSMETICS

(30) Priorité: 13.11.1995 FR 9513504
(43) Date de publication de la demande: 18.11.1998
(73) Titulaire: Crodarom S.A., 48230 Chanac (FR)
(72) Inventeur: BOUSQUET, François, F-48230 Chanac (FR)
(74) Mandataire: Laget, Jean-Loup
(86) Numéro de dépôt international: FR9601578
(87) Numéro de publication internationale: WO9718283

(56) Documents cités:
- EP-A- 0 398 798
- FR-A- 701 273
- FR-A- 2 425 241
- FR-A- 2 694 300
- FR-A- 2 723 313
- S.BUDAVARI ET AL.: "The Merck Index" 1989 , MERCK , RAHWAY,N.J. XP002020452 eleventh edition page 1375 *8680.Sorbitol*

## Description

Les extraits de plantes sont utilisés dans le domaine cosmétique ou dermopharmaceutique depuis très longtemps. Les méthodes pour obtenir un extrait sont nombreuses (macération hydroglycolique, lixiviation, décoction, cryobroyage et cytoexclusion, hydro-distillation, traitement aux solvants organiques, etc.).

Chacune des techniques a des avantages, mais aussi beaucoup d'inconvénients. A titre d'exemple, on cite le problème de la stabilisation des extraits obtenus par macération et leur très forte coloration, le coût énergétique des décoctions, les équipements lourds nécessaires pour les extractions aux solvants organiques.

Le souhait de l'industrie cosmétique aujourd'hui est multiple: on cherche des extraits de plantes pour leur "image" (produits naturels), mais on exige des produits stables (sans précipitation), incolores ou très faiblement colorés. De plus, on souhaite pouvoir mettre en évidence une ou plusieurs substances actives dans l'extrait par des méthodes d'analyses adéquates, et l'on demande à optimiser le coût de ces extraits de plantes.

Aucune des méthodes de préparation d'extraits de plantes ne répond complètement à ces exigences.

L'invention, objet du présent brevet, est la découverte que l'utilisation d'un appareil générateur de micro-ondes permet d'aboutir à satisfaire les demandes de l'industrie cosmétique telles que décrites précédemment.

Le brevet EP 0398798 décrit un procédé d'extraction de produits naturels assistée par micro-ondes. La spécificité de ce brevet est le fait que le solvant d'extraction ("extractant") est transparent ou partiellement transparent aux micro-ondes, l'argumentation étant basée sur des différences de températures générées entre l'intérieur des matériaux biologiques et le milieu externe, à savoir le solvant transparent (donc plus ou moins froid) d'extraction ; cette différence de température jouant en faveur d'une migration des substances extractables vers le solvant extérieur.

Il est également connu de FR-A-2 694 300 un procédé et un dispositif d'extraction et de fixation d'arômes dans un substrat qui est un corps gras ou de l'huile, par chauffage des parties aqueuses de plantes aromatiques sous l'action d'un rayonnement micro-ondes.

Alors que cette méthode peut s'appliquer à l'extraction d'huiles essentielles, à des substances solubles dans les solvants organiques (hexane, chloroforme etc.), elle ne convient pas aux produits cosmétiques à moins de prévoir la récupération des substances extraites par évaporation, par extraction liquide/liquide, par précipitation ou par d'autres méthodes afin d'éliminer le solvant organique inadapté à une utilisation topique. Ces opérations sont parfois complexes et augmentent considérablement le coût des extraits.

La découverte, objet du présent brevet, est le fait que l'on peut obtenir un extrait de plante par irradiation aux micro-ondes en utilisant un solvant qui absorbe fortement les micro-ondes et qui est un "solvant cosmétique", c'est-à-dire un excipient directement utilisable dans les produits cosmétiques et dermopharmaceutiques, et ceci tout en diminuant les temps d'irradiation (et donc de chauffage) dans des délais très courts (gain en coût énergétique, moins de dégradation du matériel biologique). Le résultat est un extrait qui, selon les conditions d'irradiation choisies, est peu coloré, sans trouble ni précipité, et qui contient une quantité dosable d'ingrédients actifs (saponosides, flavonoïdes, phytostérols etc.). A titre d'exemple nous comparons deux extraits obtenus l'un par décoction classique, l'autre par irradiation aux micro-ondes.

La partie aérienne de Mélisse (fleur et tige) est finement divisée. Pour la décoction, on mélange 10 g de plante avec 90 g de glycérine/eau 90/10 et on chauffe pendant une heure à 80°C. Ensuite on filtre pour obtenir un extrait initialement clair. Pour l'irradiation aux micro-ondes, on incorpore la plante dans un mélange Glycérine/Eau 90/10 (p/p) à raison de 20 g de matériel végétal pour 80 g d'extractant. Immédiatement après on irradie ce mélange dans un dispositif consistant en un tube en verre pourvu d'une vis sans fin, autour duquel sont disposés des générateurs de micro-ondes. L'irradiation s'effectue en continu sur le mélange qui traverse le tube et passe devant chacune des têtes d'émission (4 à 6 de préférence). A la sortie du tube le mélange est récolté, puis la séparation des plantes s'effectue par des moyens de filtration habituels.

L'extrait est standardisé par adjonction d'eau ou de glycérine selon la proportion finale souhaitée et pour ramener à 1/10 la proportion plante/extractant, comme dans le cas de la décoction classique.

Le dosage de l'acide rosmarinique, principe actif de la Mélisse, est effectué par spectroscopie UV et par chromatographie couche mince quantitative.

La couleur est mesurée au chromamètre Minolta.

### Résultats:

L'extrait obtenu par décoction contient 2 fois moins d'acide rosmarinique que l'extrait obtenu par micro-ondes ; il se trouble après deux jours, alors que l'extrait obtenu selon l'invention reste limpide après 3 semaines.

La couleur de l'extrait classique est 5 fois plus intense que celle obtenue par micro-ondes.

La rapidité de l'opération (1 minute d'irradiation) et le faible coût énergétique (8 kW) rendent le procédé encore plus attractif.

L'essentiel de la découverte reste néanmoins le fait que la méthode d'extraction par micro-ondes peut être appliquée sur des plantes dans un milieu non-transparent, voire un milieu qui absorbe les micro-ondes plus fortement que l'eau contenue dans les plantes. Le résultat est un extrait moins coloré, plus riche en principes actifs, plus stable et obtenu à un coût de fabrication faible. Les exigences de l'industrie cosmétique sont ainsi pratiquement satisfaites.

La méthode peut s'appliquer à toute sorte de plantes, à toute partie des plantes ou aux plantes entières. Le solvant d'extraction est obligatoirement un solvant utilisable tel quel dans le domaine cosmétique : eau, glycérine, propylène glycol, butylène glycol, polyéthylène glycol de tout poids moléculaire, monoéthyléther du diéthylène glycol, hexylène glycol et d'autres polyols (pentanediol, hexanols) ainsi que tous les mélanges de ces solvants extractants.

La proportion du matériel végétal par rapport au solvant extractant peut varier entre 1:1 à 1:20 (p/p). La durée d'irradiation peut varier entre 10 et 300 secondes, préférentiellement entre 30 et 100 secondes. La puissance des générateurs de micro-ondes peut varier entre 100 et 2000 Watts, la fréquence d'irradiation étant limitée entre 2000 et 3000 Mhz.

Les extraits ainsi obtenus peuvent être utilisés dans toute forme galénique employée en cosmétique : émulsions H/E et E/H, laits, lotions, gels, pommades, shampooings et après-shampooings, savons, laques pour cheveux.

Il est possible d'incorporer les extraits obtenus par micro-ondes décrits dans des vecteurs cosmétiques comme les liposomes, les chylomicrons, les macro-, micro-et nanoparticules ainsi que les macro-, micro- et nanocapsules ; on peut les adsorber sur des polymères organiques poudreux, les talcs, bentonites et autres supports minéraux.

Les extraits obtenus par micro-ondes, objets du présent brevet, peuvent être combinés dans les compositions cosmétiques avec tout autre ingrédient habituellement utilisé en cosmétique : lipides d'extraction et ou de synthèse, polymères gélifiants et viscosants, tensioactifs et émulsifiants, principes actifs hydro- ou liposolubles, extraits d'autres plantes, extraits tissulaires, extraits marins. Les compositions cosmétiques contenant les extraits obtenus par micro-ondes, objets du présent brevet, sont destinées à toutes les applications cosmétiques à savoir, soins et hygiène de la peau, du cuir chevelu, des cheveux, des muqueuses, des régions bucco-dentaires, pour les traitements anti-vieillissement et la protection solaire, pour l'hydratation ou l'effet de lissage.

## Revendications

1. Procédé d'extraction de plantes pour la fabrication d'extraits consistant en l'irradiation par micro-ondes d'un mélange de plantes et d'un ou plusieurs solvants d'extraction, **caractérisé en ce que** le(s) solvants(s) d'extraction absorbe(nt) fortement les micro-ondes et est(sont) choisi(s) parmi les solvants suivants, directement utilisables dans les produits cosmétiques et dermopharmaceutiques: eau, glycérine, propylène glycol, butylène glycol, polyéthylène glycol de tout poids moléculaire, monoéthyléther du diéthylène glycol, hexylène glycol et autres polyols (pentanediol, hexanols) et tous mélanges de ces solvants.

2. Procédé d'extraction de plantes selon la revendication 1, **caractérisé en ce que** la proportion du matériel végétal par rapport au solvant extractant varie entre 1:1 et 1:20 (poids/poids), que la durée d'irradiation aux micro-ondes varie entre 10 et 300 secondes, préférentiellement entre 30 et 100 secondes, et que la puissance des générateurs de micro-ondes varie entre 100 et 2000 Watts, la fréquence d'irradiation étant limitée entre 2000 et 3000 Mhz.

3. Utilisation des extraits obtenus par micro-ondes par le procédé selon les revendications 1 et 2 dans toute forme galénique employée en cosmétique, notamment, émulsions H/E et E/H, laits, lotions, gels, pommades, shampooings et après-shampooings, savons, laques pour cheveux.

4. Utilisation selon la revendication 3 **caractérisée en ce que** les extraits obtenus par micro-ondes sont incorporés dans ces vecteurs cosmétiques comme les liposomes, les chylomicrons, les macro-, micro- et nanoparticules ainsi que les macro-, micro- et nanocapsules ou absorbés sur des polymères organiques poudreux, les talcs, bentonites et autres supports minéraux.

5. Utilisation selon la revendication 3 ou 4 **caractérisée en ce que** les extraits obtenus par micro-ondes, sont combinés dans les compositions cosmétiques avec tout autre ingrédient habituellement utilisé en cosmétique: lipides d'extraction et ou de synthèse, polymères gélifiants et viscosants, tensioactifs et émulsifiants, principes actifs hydro- ou liposolubles, extraits d'autres plantes, extraits tissulaires, extraits marins.

6. Utilisation des extraits obtenus par micro-ondes par le procédé selon les revendications 1 ou 2 dans toutes les applications cosmétiques à savoir, notamment, soins ou hygiène de la peau, du cuir chevelu, des cheveux, des muqueuses, des régions bucco-dentaires, pour les traitements anti-vieillissement et la protection solaire, pour l'hydratation, l'effet de lissage.

## Patentansprüche

1. Verfahren zur Extraktion von Pflanzen zur Herstellung von Extrakten, das darin besteht daß man ein Gemisch von Pflanzen und einem oder mehreren Extraktionslösungsmitteln mit Mikrowellen bestrahlt, **dadurch gekennzeichnet, daß** das bzw. die Extraktionslösungsmittel die Mikrowellen stark absorbiert bzw. absorbieren und aus der Gruppe der folgenden Lösungsmittel, die direkt in Kosmetikprodukten und Hautpharmazeutika verwendet werden können, ausgewählt ist bzw. sind: Wasser, Glyzerin, Propylenglykol, Butylenglykol, Polyethylenglykol mit einem beliebigen Molekulargewicht, Diethylenglykolmonoethylether, Hexylenglykolmonoethylether und Ethylether anderer Polyole (Pentandiol, Hexanole) sowie alle Mischungen dieser Lösungsmittel.

2. Verfahren zur Extraktion von Pflanzen nach Anspruch 1, **dadurch gekennzeichnet, daß** das Verhältnis zwischen Pflanzenmaterial und Extraktionslösungsmittel zwischen 1:1 und 1:20 (Gew./Gew.) liegt, daß die Dauer der Mikrowellenbestrahlung zwischen 10 und 300 Sekunden, vorzugsweise zwischen 30 und 100 Sekunden, liegt und daß die Leistung der Mikrowellenquelle zwischen 100 und 2000 Watt liegt, wobei die Bestrahlungsfrequenz auf zwischen 2000 und 3000 MHz beschränkt ist.

3. Verwendung von nach dem Verfahren nach den Ansprüchen 1 und 2 mittels Mikrowellen gewonnenen Extrakten in beliebigen in der Kosmetik gebräuchlichen galenischen Formen, insbesondere in Form von O/W- und W/O-Emulsionen, Milchen, Lotionen, Gelen, Pomaden, Shampoos, Mitteln zur Verwendung nach der Haarwäsche, Seifen, Haarlacken.

4. Verwendung nach Anspruch 3, **dadurch gekennzeichnet, daß** die mittels Mikrowellen gewonnenen Extrakte in kosmetische Vehikel wie Liposomen, Chylomikronen, Makro-, Mikro- und Nanopartikel sowie Makro-, Mikro- und Nanokapseln verpackt bzw. auf pulverförmige organische Polymere, verschiedene Arten von Talk, Bentonite und andere mineralische Träger aufgezogen werden.

5. Verwendung nach Anspruch 3 oder 4, **dadurch gekennzeichnet, daß** die mittels Mikrowellen gewonnenen Extrakte in den Kosmetikzusammensetzungen mit beliebigen weiteren in der Kosmetik gebräuchlichen Bestandteilen vereinigt werden: Extraktions- oder Syntheselipiden, Gelbildner- und Verdickungs-, Tensid- und Emulgatorpolymeren, wasser-oder lipidlöslichen Wirkstoffen, Extrakten aus anderen Pflanzen, Gewebeextrakten, marinen Extrakten.

6. Verwendung von nach dem Verfahren nach den Ansprüchen 1 oder 2 mittels Mikrowellen gewonnenen Extrakten in beliebigen kosmetischen Anwendungen, nämlich insbesondere in der Pflege oder Hygiene der Haut, der Kopfhaut, des Haars, der Schleimhäute, des Mund- und Zahnbereichs, zur Anwendung gegen das Altern und als Sonnenschutz, als Feuchtigkeitsspender sowie zur Glättung.

## Claims

1. A plant extraction process for producing extracts, consisting of the microwave irradiation of a mixture of plants and one or more extraction solvents, **characterised in that** the extraction solvent(s) absorb(s) the microwaves to a great extent and is(are) selected from among the following solvents, which can be used directly in cosmetic and dermatological products: water, glycerine, propylene glycol, butylene glycol, polyethylene glycol of any molecular weight, diethylene glycol monoethyl ether, hexylene glycol and other polyols (pentanediol, hexanols) and any mixtures of these solvents.

2. A plant extraction process according to Claim 1, **characterised in that** the proportion of the plant material relative to the extracting solvent varies from 1:1 to 1:20 (weight/weight), that the duration of microwave irradiation varies between 10 and 300 seconds, preferably between 30 and 100 seconds, and that the power of the microwave generators varies between 100 and 2000 watt, the irradiation frequency being limited to between 2000 and 3000 MHz.

3. The use of the extracts obtained by microwaves by the process according to Claims 1 and 2 in any pharmaceutical form used in cosmetics, in particular O/W and W/O emulsions, milks, lotions, gels, creams, shampoos and conditioners, soaps and hairsprays.

4. The use according to Claim 3, **characterised in that** the extracts obtained by microwaves are incorporated into these cosmetic carriers such as liposomes, chylomicrons, macro-, micro- and nanoparticles and also macro-, micro and nanocapsules, or absorbed on powdered organic polymers, talcs, bentonites and other mineral supports.

5. The use according to Claim 3 or 4, **characterised in that** the extracts obtained by microwaves are combined in the cosmetic compositions with any other ingredient commonly used in the field of cosmetics: extraction and/or synthesis lipids, gelling and thickening polymers, surfactants and emulsifiers, water- or fat-soluble active ingredients, extracts of other plants, tissue extracts or marine extracts.

6. The use of the extracts obtained by microwaves by the process according to Claims 1 or 2 in all cosmetic applications, namely skin, scalp and hair care or hygiene, oral and dental care or hygiene, for anti-ageing and sun-protection treatments, for moisturising and smoothing effects.
